# EUROPEAN PATENT SPECIFICATION

(11) **EP 3 829 533 B1**
(45) Date of publication and mention of the grant of the patent: **08.05.2024**
(21) Application number: 18755694.9
(22) Date of filing: 31.07.2018
(51) Int. Cl.: A61K 8/73, A61K 8/46, A61K 8/29, A61K 8/19, A61K 8/40

(54) **SUNSCREEN COMPOSITION, USE OF THE SUNSCREEN COMPOSITION AND PROCESS OF MANUFACTURING THE SUNSCREEN COMPOSITION**
SONNENSCHUTZMITTEL, VERWENDUNG DES SONNENSCHUTZMITTELS UND VERFAHREN ZUR HERSTELLUNG DER SONNENSCHUTZMITTEL
COMPOSITION D'ÉCRAN SOLAIRE, UTILISATION DE LA COMPOSITION D'ÉCRAN SOLAIRE ET PROCÉDÉ DE FABRICATION DE LA COMPOSITION D'ÉCRAN SOLAIRE

(43) Date of publication of application: 09.06.2021
(73) Proprietor: L'OREAL, 75008 Paris (FR); Trajano, Patricia, 21941-972 Rio De Janeiro-RJ (BR)
(72) Inventor: TRAJANO, Patricia, 21941-972 Rio De Janeiro-RJ (BR); FOLLONA-MORENO, Angeles, 21941-972 Rio De Janeiro-RJ (BR); FERREIRA, Renata Souto Maior Afonso, 21941-972 Rio De Janeiro-RJ (BR); HANASHIRO, Tassia, 21941-972 Rio De Janeiro-RJ (BR); BARCELOS, José Mario Melo, 21941-972 Rio De Janeiro-RJ (BR); CAMARGO, Ludmila Pons, 21941-972 Rio De Janeiro-RJ (BR)
(74) Representative: Casalonga
(86) International application number: PCT/BR2018/050266
(87) International publication number: WO 2020/024023

(56) References cited:
- WO-A1-2017/029807
- WO-A1-2017/030209

## Description

### FIELD OF THE INVENTION

The present invention is directed to new sunscreen compositions with high SPF, the process of manufacturing the composition and its uses.

### BACKGROUND OF THE INVENTION

The photoprotection of keratinous materials, including both skin and hair, is considered of great importance in order to protect from sun-damage, sunburn, photo-aging, as well as to decrease the chances of skin cancer development caused by exposure to ultraviolet ("UV") radiation. There are typically two types of UVA/UVB sunscreen compositions used to accomplish photoprotection, namely, inorganic UV filters and organic UV filters.

The degree of UV protection afforded by a sunscreen composition is directly related to the amount and type of UV filters contained therein. The higher the amount of UV filters, the greater the degree of UV protection (UVA/UVB).

Particularly, sunscreen compositions must provide good protection against the sun, a measure of which is the Sun Protection Factor (SPF) value, yet have satisfactory sensory perception, such as a smooth but not greasy feel upon application. However, this combination of properties has been difficult to achieve, particularly because many active sunscreen compounds themselves have an oily or greasy feel, and increasing their content tends to cause the final product to suffer from that effect.

Also, most organic sunscreen filters are oil-like and/or oil-soluble materials. High levels of sunscreen filters in sunscreen products render the products less appealing for their greasy skin feel, stickiness, long drying time, and leave shiny residue on the skin after application. The filters also have the tendency to whiten the skin after application and after becoming wet with water or perspiration which is an undesirable attribute.

Additionally, an important problem of sunscreen compositions is that due to the great amount of sunscreen filters associated with great amount of fillers to ensure the good sensoriality and pigments in the O/W emulsion, it tends to be unstable.

Therefore, a stable sunscreen composition with high SPF is desired, associated with easy application, good spreadability, less white film, less shine, which does not melt at high temperature on the face, has an imperceptible touch, gives the ideal balance between hydration and oil control and combines high protection with smoothness to the skin.

The challenge of formulating a sunscreen composition having high SPF associated with the unique sensorial described above is that such composition uses high amounts of essential ingredients (association of UV filters, fillers and pigments) and tends to be unstable and ineffective.

The pigments are essential raw materials for delivering an additional attribute to the sunscreen composition, which is the uniformization of the skin tone. However, such association of pigments to a sunscreen composition having high SPF results in a high complexity challenge to stabilize the emulsion.

Thus, the inventors succeeded to overcome the problems of the state of the art and surprisingly revealed a stable sunscreen composition with high SPF, light feel and pleasant sensorial through a specific a combination of at least one anionic surfactant and one non-ionic co-surfactant, UV filters and pigments.

### SUMMARY OF THE INVENTION

The present invention is defined in the asppended claims and is directed to new sunscreen compositions with high SPF, comprising (a) a combination of at least one anionic surfactant and one non-ionic co-surfactant, (b) UV filters and (c) pigments.

The composition of the present invention is stable over the time, also presents a high level of UV-protection in order to protect the skin from the damages of the sun, easy application, good spreadability, less white film, less shine, which does not melt on high temperature in the face, has an imperceptible touch and combines high protection with smoothness to the skin.

### BRIEF DESCRIPTION OF THE DRAWINGS

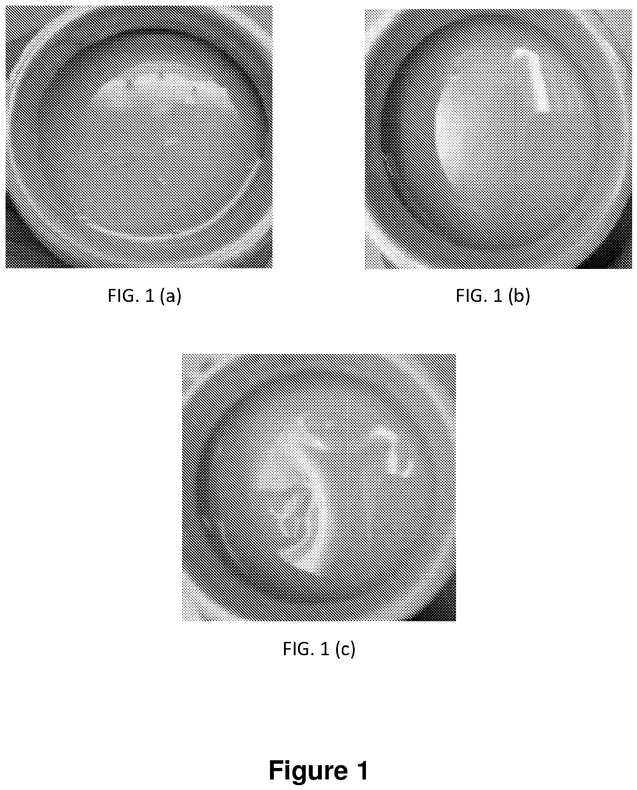

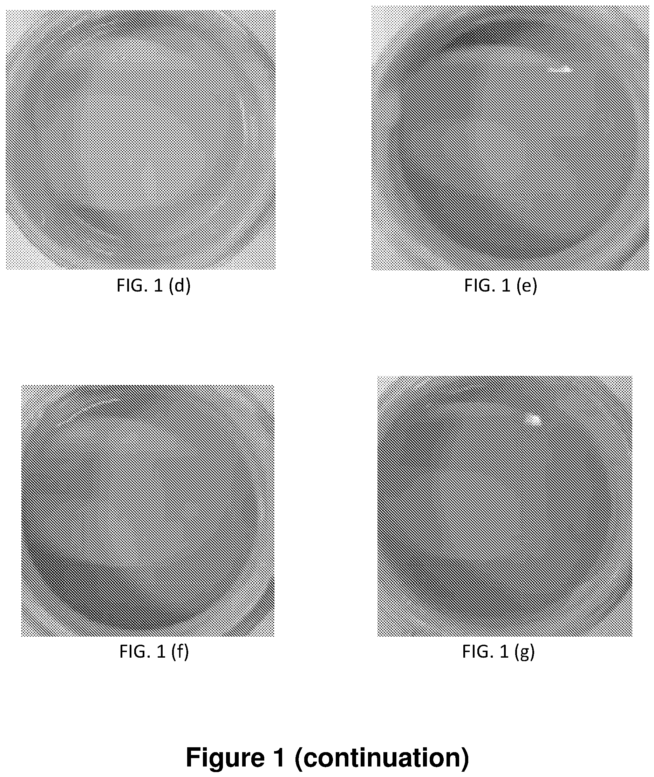

FIG. 1 (a) to (g) are photographs of the visual aspect of the sunscreen composition of the present invention, according to Example 2, demonstrating the stability of the ingredients, after 30 days at 20ºC, 37ºC and 45ºC (FIG. 1 (a) to (c), respectively), after 60 days at 20ºC, 37ºC and 45ºC (FIG. 1 (d) to (f), respectively) and after a sun test (FIG. 1 (g)).

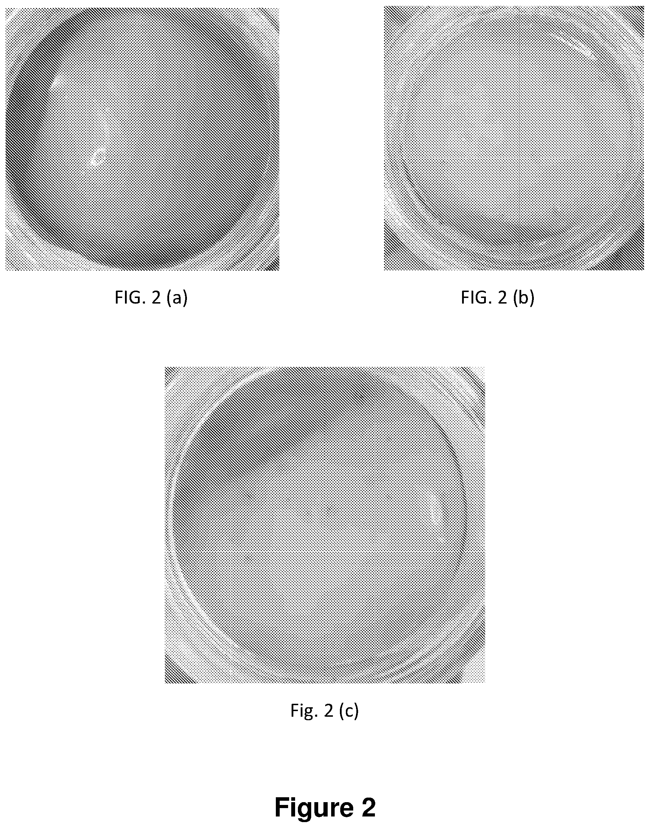

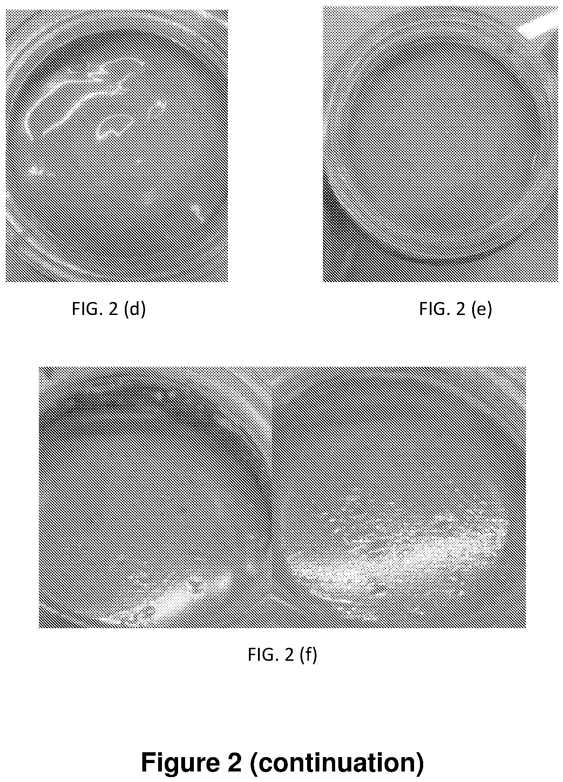

FIG. 2 (a) to (f) are photographs of the visual aspect of the sunscreen composition of the prior art, according to Example 1, demonstrating the instability of the ingredients, after 30 days at 20ºC, 37ºC, 45ºC (FIG. 2 (a) to (c), respectively) and after 60 days at 20ºC, 37ºC, 45ºC (FIG. 2 (d) to (f), respectively).

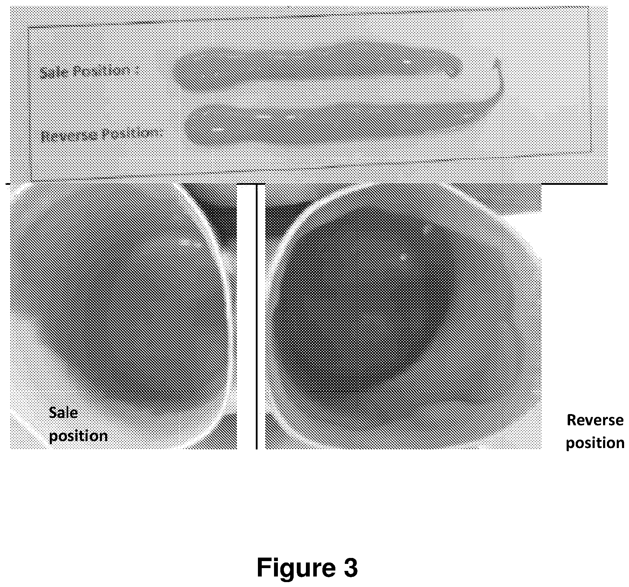

FIG. 3 are photographs of the visual aspect of the sunscreen composition of the present invention, according to Example 2, demonstrating the superior and robust compatibility of the O/W emulsion over 2 months, at 45 ºC, inside the final package of the product, at the top (sale position) and at the bottom (reverse position) of the package.

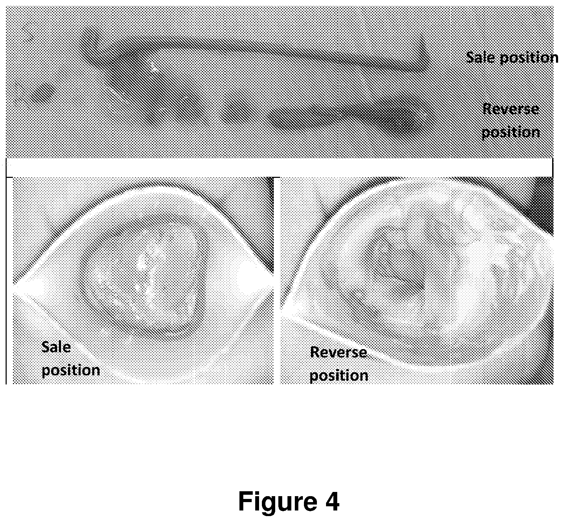

FIG. 4 are photographs of the visual aspect of the sunscreen composition of Example 1 (prior art), demonstrating the poor compatibility of the ingredients inside the final package of the O/W emulsion over 2 months, at 45 ºC, at the top (sale position) and at the bottom (reverse position) of the package.

### DETAILED DESCRIPTION OF THE INVENTION

In an embodiment, the sunscreen composition of the present invention comprises:
(a) a combination of at least one anionic surfactant and one non-ionic co-surfactant selected from inulin lauryl carbamate and sodium N-stearoyl-N-methyl taurate, respectively;
(b) UV filters; and
(c) pigments.

The composition according to the invention provides excellent sensorial performance as well as surprisingly high SPF values associated with a strong and robust stability of the composition over the time.

The amount of the combination of at least one anionic surfactant and one non-ionic co-surfactant is ranging from about 0.5% to 10% by weight and preferably from about 1.5% to about 8% by weight, or more preferably from about 3% to about 6% by weight, relative to the total weight of the composition.

The combination of at least one anionic surfactant and one non-ionic co-surfactant employs a surfactant in an amount preferably ranging from about 0.01% to about 7% by weight and preferably from about 0.1% to about 5% by weight, or more preferably from about 0.2% to about 3% by relative to the total weight of the composition.

In a preferred embodiment, the combination of at least one anionic surfactant and one non-ionic co-surfactant employs a co-surfactant in an amount preferably ranging from about 0.1% to about 7% by weight and preferably from about 0.5 to about 6% by weight, or more preferably from about 1% to about 3% by weight, relative to the total weight of the composition.

The UV filters used in the composition of the present invention are selected from the group of oil-soluble organic sunscreen ingredients, water-soluble organic sunscreen ingredients and silica-coated titanium dioxide sunscreen ingredients.

The amount of the UV filters in the sunscreen composition of the invention ranges from about 3% to about 50% by weight, preferably in an amount of from about 5% to about 40% by weight, and most preferably about 7% to about 30% by weight, based on the total weight of the composition.

In a preferred embodiment, the pigments used in the sunscreen composition of the present invention are selected from the group of metal oxide pigments, such as titanium dioxide, zinc oxide, titanium oxide, iron oxide, zirconium oxide and cerium oxide, or mixtures thereof.

The amount of the pigments in the sunscreen composition of the invention ranges from about 0.01 % to about 15% by weight, and most preferably about 0.5% to about 10% by weight, based on the total weight of the composition.

The pH of the sunscreen composition of the invention is preferably within the range of about 5.5 to about 7.0, more preferably, of about 6.0 to about 6.5.

In a preferred embodiment, the sunscreen composition of the present invention may present a Sun Protection Factor ranging from 30 to 80.

In various embodiments, the sunscreen composition of the present invention may present a Sun Protection Factor of 30, 35, 40, 45, 50, 55, 60, 65, 70 and 80.

In an embodiment, the sunscreen composition of the present invention may present a Sun Protection Factor of 50.

In an embodiment, the sunscreen composition of the present invention may present a Sun Protection Factor of 60.

In an embodiment, the sunscreen composition of the present invention may present a Sun Protection Factor of 70.

In an embodiment, the sunscreen composition of the present invention may present a Sun Protection Factor of 80.

The sunscreen composition of the invention is in the form of an oil in water (O/W) emulsion.

The sunscreen composition of the invention can be used as a daily product for the skin.

In addition, the sunscreen composition according to the present invention is cutaneous safe.

The composition of the present invention presents a high level of UV-protection in order to protect the skin from the damages of the sun, easy application, good spreadability, less white film, less shine, which does not melt on high temperature in the face, has an imperceptible touch and combines high protection with smoothness to the skin. Also, the composition of the present invention is stable over the time.

In another preferred embodiment, the present invention is related to the use of a composition for manufacturing a product for preventing sunburn.

### Terms

As used herein, the expression "at least" means one or more and thus includes individual components as well as mixtures/combinations.

Other than in the operating examples, or where otherwise indicated, all numbers expressing quantities of ingredients and/or reaction conditions are to be understood as being modified in all instances by the term "about," meaning within +/-5% of the indicated number.

As used herein, all ranges provided are meant to include every specific range within, and combination of sub ranges between, the given ranges. Thus, a range from 1-5, includes specifically 1, 2, 3, 4 and 5, as well as sub ranges such as 2-5, 3-5, 2-3, 2-4, 1-4, etc. All ranges and values disclosed herein are inclusive and combinable. For examples, any value or point described herein that falls within a range described herein can serve as a minimum or maximum value to derive a sub-range, etc.

### Additional Surfactants

The suitable combination of at least one anionic surfactant and one non-ionic co-surfactant of the present invention may comprise additional surfactants that could be chosen among the surfactants described below.

Anionic surfactants useful in the invention include, for example, carboxylates (sodium 2-(2-hydroxyalkyloxy)acetate), amino acid derivatives (N-acylglutamates, N-acylglycinates or acylsarcosinates), alkyl sulfates, alkyl ether sulfates and oxyethylenated derivatives thereof, sulfonates, isethionates and N-acylisethionates, taurates and N-acyl N-methyltaurates, sulfosuccinates, alkyl sulfoacetates, phosphates and alkyl phosphates, anionic derivatives of alkyl polyglycoside (acyl-D-galactoside uronate), and mixtures thereof.

Non-limiting examples of non-ionic surfactants useful in the invention include, for example, oxyalkylenated (more particularly polyoxyethylenated) fatty acid esters of glycerol; oxyalkylenated fatty acid esters of sorbitan; oxyalkylenated (oxyethylenated and/or oxypropylenated) fatty acid esters; oxyalkylenated (oxyethylenated and/or oxypropylenated) fatty alcohol ethers; sugar esters, for instance sucrose stearate; fatty alcohol ethers of sugars, especially alkyl polyglucosides (APGs) such as decyl glucoside and lauryl glucoside, cetostearyl glucoside optionally as a mixture with cetostearyl alcohol, and also arachidyl glucoside, for example in the form of a mixture of arachidyl alcohol, behenyl alcohol and arachidyl glucoside. According to one particular embodiment of the invention, the mixture of the alkyl polyglucoside as defined above with the corresponding fatty alcohol may be in the form of a self-emulsifying composition. Mention may also be made of lecithins and derivatives (e.g. Biophilic), sugar esters and sodium stearoyl lactylate.

Non-limiting examples of fatty acids chosen from C_{12-C22} higher fatty acids, such as myristic acid, oleic acid, linoleic acid, linolenic acid, lauric acid, palmitic acid, and mixtures thereof.

The additional surfactants may present in the sunscreen composition of the present invention in an amount of preferably ranging from about 0.1 to about 10% by weight and preferably from about 0.3 to about 5% by weight, or more preferably from about 0.5 to about 3% by weight, including all ranges and sub-ranges there between, relative to the total weight of the composition.

### UV Filters

Non-limiting suitable UV filters of the present invention could be as follows:

### Oil-soluble organic sunscreen ingredient

The "oil-soluble organic sunscreen ingredient" means any organic compound for screening out UV radiation, which can be fully dissolved in molecular form or miscible in an oil phase or which can be dissolved in colloidal form (for example in micellar form) in an oil fatty phase.

Non-limiting examples of oil-soluble organic sunscreen ingredients useful in the invention include, for example, cinnamic derivatives; anthranilates; salicylic derivatives; dibenzoylmethane derivatives; camphor derivatives; benzophenone derivatives; diphenylacrylate derivatives; triazine derivatives; benzotriazole derivatives; benzalmalonate derivatives, especially those cited in patent US5624663; benzimidazole derivatives; imidazolines; bis-benzoazolyl derivatives as described in patents EP669323 and US2463264; p-aminobenzoic acid (PABA) derivatives; methylene bis(hydroxyphenylbenzotriazole) derivatives as described in applications US5237071, US5166355, GB2303549, DE19726184 and EP893119; benzoxazole derivatives as described in patent applications EP0832642, EP1027883, EP1300137 and DE10162844; screening polymers and screening silicones such as those described especially in patent application WO 93/04665; dimers derived from alkyl-styrene such as those described in patent application DE 19855649; 4,4-diarylbutadienes such as those described in patent applications EP0967200, DE19746654, DE19755649, EP-A-1008586, EP1133980 and EP1133981, merocyanine derivatives such as those described in patent applications WO 04/006878, WO 05/058269 and WO 06/032741; and mixtures thereof, the entire contents of the patents and patent applications being incorporated by reference in their entirety.

As examples of other suitable oil-soluble organic sunscreen ingredients, mention may be made of those denoted herein below under their INCI name:

### Cinnamic derivatives:

Examples of suitable cinnamic derivatives include, but are not limited to, ethylhexyl methoxycinnamate, isopropyl methoxycinnamate, isoamyl methoxycinnamate, DEA methoxycinnamate, diisopropyl methylcinnamate, glyceryl ethylhexanoate dimethoxycinnamate.

### Dibenzoylmethane derivatives:

Examples of suitable dibenzoylmethane derivatives include, but are not limited to, butyl methoxydibenzoylmethane and isopropyl dibenzoylmethane.

### Salicylic derivatives:

Examples of suitable salicylic derivatives include, but are not limited to, homosalate, ethylhexyl salicylate, dipropylene glycol salicylate and TEA salicylate.

### Beta, beta -Diphenylacrylate derivatives:

Examples of suitable beta, beta -diphenylacrylate derivatives include, but are not limited to, octocrylene and etocrylene.

### Benzophenone derivatives:

Examples of suitable benzophenone derivatives include, but are not limited to, benzophenone-1, benzophenone-2, benzophenone-3 or oxybenzone, benzophenone-4, benzophenone-5, benzophenone-6, benzophenone-8, benzophenone-9, benzophenone-12, n-hexyl 2-(4-diethylamino-2-hydroxybenzoyl)benzoate +" or as a mixture with octyl methoxycinnamate.

### Benzylidenecamphor derivatives:

Examples of suitable benzylidenecamphor derivatives include, but are not limited to, 3-benzylidene camphor manufactured, 4-methylbenzylidene camphor, polyacrylamidomethyl benzylidene camphor manufactured.

### Phenylbenzotriazole derivatives:

Examples of suitable phenylbenzotriazole derivatives include, but are not limited to, drometrizole trisiloxane, methylene bis-benzotriazolyl tetramethylbutylphenol, or in micronized form as an aqueous dispersion.

### Triazine derivatives:

Examples of suitable triazine derivatives include, but are not limited to, bis-ethylhexyloxyphenol methoxyphenyl triazine, ethylhexyl triazone, diethylhexyl butamido triazone, 2,4,6-tris(dineopentyl 4'-aminobenzalmalonate)-s-triazine, 2,4,6-tris(diisobutyl 4'-aminobenzalmalonate)¬s triazine, 2,4-bis(dineopentyl 4'-aminobenzalmalonate)-6-(n-butyl 4'-aminobenzoate)-s-triazine, symmetrical triazine screening agents described in patent US 6,225,467, patent application WO 2004/085412 (see compounds 6 and 9) or the document "Symmetrical Triazine Derivatives" IP.COM Journal, IP.COM Inc., West Henrietta, NY, US (20 September 2004), especially 2,4,6-tris(biphenyl)-1,3,5-triazines (in particular 2,4,6-tris(biphenyl-4-yl)-1,3,5-triazine and 2,4,6-tris(terphenyl)-1,3,5-triazine, which is included in patent applications WO 06/035000, WO 06/034982, WO 06/034991, WO 06/035007, WO 2006/034992 and WO 2006/034985).

### Anthranilic derivatives:

An example of a suitable anthranilic derivative includes, but is not limited to, methyl anthranilate.

### Imidazoline derivatives:

An example of a suitable imidazoline derivative includes, but is not limited to, ethylhexyl dimethoxybenzylidene dioxoimidazoline propionate.

### Benzalmalonate derivatives:

An example of a suitable benzalmalonate derivative includes, but is not limited to, polyorganosiloxane containing benzalmalonate functions, for instance polysilicone-15.

### 4,4-Diarylbutadiene derivatives:

An examples of a suitable 4,4-diarylbutadiene derivative includes, but is not limited to, 1-Dicarboxy(2,2'-dimethylpropyl)-4,4-diphenyl-butadiene.

### Benzoxazole derivatives:

An example of suitable benzoxazole derivative includes, but is not limited to, 2,4-bis[5-(1-dimethylpropyl)benzoxazol-2-yl-(4-phenyl)imino]-6-(2-ethylhexyl) imino-1 ,3,5-triazine, and mixtures thereof.

Preferably, the oil-soluble organic sunscreen ingredient will be chosen from butyl methoxydibenzoylmethane, ethylhexyl salicylate, ethylhexyl triazone, octocrylene, drometrizole trisiloxane, bis-ethylhexyloxyphenol methoxyphenyl triazine, and mixtures thereof.

The oil-soluble organic sunscreen ingredient is preferably present in the composition according to the invention in an amount of from about 3% to about 25% by weight, preferably in an amount of from about 5% to about 20% by weight, and most preferably about 7% to about 18% by weight, based on the total weight of the composition.

### Water-soluble organic sunscreen ingredient

The "water-soluble organic sunscreen ingredient" means any organic compound for screening out UV radiation, which can be fully dissolved in molecular form or miscible in a liquid aqueous phase or which can be dissolved in colloidal form (for example in micellar form) in a liquid aqueous phase.

Non-limiting examples of water-soluble organic sunscreen ingredients useful in the invention include, for example, terephthalylidene dicamphor sulfonic acid, phenylbenzimidazole sulfonic acid, benzophenone-4, aminobenzoic acid (PABA), 4-Bis(polyethoxy)-para-aminobenzoic acid polyethoxyethyl ester (PEG-25 PABA), camphor benzalkonium methosulfate, methylene bis-benzotriazolyl tetramethylbutylphenol (Bisoctrizole), disodium phenyl dibenzimidazole tetrasulfonate (Bisdisulizole disodium), and tris-biphenyl triazine; their derivatives and corresponding salts; naphthalene bisimide derivatives such as those described in European patent application EP1990372 A2, the entire contents of which is hereby incorporated by reference; and cinnamido amine cationic quaternary salts and derivatives such as those described in United States Patent 5,601,811, the entire contents of which is hereby incorporated by reference, and mixtures thereof.

The salts of the compounds that may be used according to the invention are chosen in particular from salts of alkali metals, for example sodium or potassium; salts of alkaline-earth metals, for example calcium, magnesium or strontium; metal salts, for example zinc, aluminum, manganese or copper; salts of ammonium of formula NH4+; quaternary ammonium salts; salts of organic amines, for instance salts of methylamine, dimethylamine, trimethylamine, triethylamine, ethylamine, 2-hydroxyethylamine, bis(2-hydroxyethyl)amine or tris(2-hydroxyethyl)amine; lysine or arginine salts. Salts chosen from sodium, potassium, magnesium, strontium, copper, manganese or zinc salts are preferably used. The sodium salt is preferably used.

Preferably, the water-soluble organic sunscreen ingredient will be chosen from terephthalylidene dicamphor sulfonic acid, methylene bis-benzotriazolyl tetramethylbutylphenol, and mixtures thereof.

The water-soluble organic sunscreen ingredient is preferably present in the composition according to the invention in an amount of from about 0.1% to about 10% by weight, preferably in an amount of from about 0.5% to about 8% by weight, and most preferably about 1% to about 7% by weight, based on the total weight of the composition.

### Silica-coated titanium dioxide sunscreen ingredient

The "silica-coated titanium dioxide sunscreen ingredient" means spherical beads which are formed by encapsulating titanium dioxide particles in silica.

Non-limiting examples of silica coated titanium dioxide sunscreen ingredients useful in the invention include, for example, titanium dioxide coated with silica, such as name silica (and) titanium dioxide having a composition of silica: titanium dioxide of about 55:45 and having a particle size from about 2 microns to about 7 microns.

The silica-coated titanium dioxide sunscreen ingredient is preferably present in the composition according to the invention in an amount of from about 1% to about 10% by weight, preferably in an amount of from about 2% to about 10% by weight, and most preferably about 5% to about 10% by weight, based on the total weight of the composition.

The suitable UV filter system of the present invention comprises terephthalylidene dicamphor sulfonic acid, octocrylene and butyl methoxydibenzoylmethane sulfonic acid.

### PIGMENTS

The suitable pigments used in the sunscreen composition of the present invention may be coated or uncoated.

The coated pigments are pigments which have undergone one or more surface treatments of a chemical, electronic, mechanochemical and/or mechanical nature with compounds such as those described for example in Cosmetics & Toiletries, February 1990, vol. 105, p.53-64, such as amino acids, beeswax, fatty acids, fatty alcohols, anionic surface-active agents, lecithin, sodium, potassium, zinc, iron or aluminum salts of fatty acids, (titanium or aluminum) metal alkoxides, polyethylene, silicones, proteins (collagen, elastin), alkanolamines, silicon oxides, metal oxides or sodium hexametaphosphate.

Coated pigments are more particularly titanium oxides coated with silica such as the product, silica and iron oxide, silica and alumina, alumina such as the products, alumina and aluminum stearate, alumina and aluminum laurate, iron oxide and iron stearate, zinc oxide and zinc stearate, silica, alumina and silicone, silica, alumina, aluminum stearate and silicone, alumina and silicone, etc.

Mixtures of metal oxides may also be mentioned, especially titanium dioxide and cerium dioxide, including the silica-coated equiponderous mixture of titanium dioxide and cerium dioxide, as well as the alumina-silica- and silicone-coated mixture of titanium oxide and zinc dioxide, or the alumina-, silica- and glycerin-coated mixture of titanium dioxide and zinc dioxide.

In addition, uncoated titanium oxides, zinc oxides and cerium oxide may be used in the sunscreen composition of the present invention.

### ADDITIONAL INGREDIENTS

In addition to the essential components described hereinbefore, the composition of the invention may further comprise any usual cosmetically acceptable ingredient, which may be chosen especially from such as additional sunscreens, perfume/fragrance, preserving agents, solvents, actives, surfactants, fatty compounds, vitamins, fillers, silicones, polymers, pigments and mixtures thereof.

A person skilled in the art will take care to select the optional additional ingredients and/or the amount thereof such that the advantageous properties of the composition according to the invention are not, or are not substantially, adversely affected by the envisaged addition.

Non-limiting example of preserving agent which can be used in accordance with the invention include phenoxyethanol.

Suitable fillers of the invention could be as examples of oil-absorbing fillers: mica, silica, zea may (corn) starch, magnesium oxide, nylon-12, nylon-66, cellulose, polyethylene, talc, talc (and) methicone, talc (and) dimethicone, perlite, sodium silicate, pumice, PTFE, polymethyl methacrylate, oryza sativa (rice) starch, aluminum starch octenylsuccinate, potato starch modified, alumina, silica silylate, calcium sodium borosilicate, magnesium carbonate, hydrated silica, dimethicone/vinyl dimethicone crosspolymer, sodium carboxylmethyl starch.

Suitable solvents include, but are not limited to water, alcohols, glycols and polyols such as glycerin, water, caprylyl glycol, pentylene glycol, propylene glycol, butylene glycol, C₁₂₋₁₅ alkyl benzoate and mixtures thereof.

In various embodiments, the solvent is present in a concentration from about 15 to 100% by weight, or from about 20 to about 80% by weight, or from about 30 to about 70% by weight, or from about 35 to about 75% by weight, or preferably from about 40 to about 70% by weight, and more preferably from about 45 to about 65% by weight, including ranges and sub-ranges there between, based on the total weight of the combinations and/or compositions of the present disclosure.

Suitable additional actives include, but are not limited to, disodium EDTA, triethanolamine, and mixtures thereof.

Non-limiting examples of additional surfactants suitable for use are fatty acids, glyceryl esters in addition to glyceryl stearate, alkoxylated fatty alcohols, such as stearic acid, laureth-12, glyceryl isostearate, disodium stearoyl glutamate, potassium cetyl phosphate, poloxamer 338, sodium methyl stearoyl taurate and mixtures thereof.

Exemplary of fat or oil materials include, but are not limited to, esters, fatty acids, synthetic oils, and hydrocarbons/paraffins, such as stearyl alcohol, myristic acid, palmitic acid. silicones mineral oil, plant/vegetable oils, and mixtures thereof.

Non-limiting example of vitamins suitable for the composition of the present invention includes tocopherol.

Examples of silicones used in the composition of the present invention but not limited to are dimethicone and caprylyl methicone.

Exemplary of polymers, include, but not limited to, aluminum starch octenylsuccinate, xanthan gam, acrylates/C₁₀-₃₀ alkyl acrylate crosspolymer and styrene/acrylates copolymer.

The additional ingredients may represent from 60% to 85%, such as from 60% to 82% or such as from 65 to 80% by weight of the total weight of the composition of the invention.

The invention will now be described with reference to the following examples.

### EXAMPLES

### Examples 1 to 4

A suitable composition according to the state of the art is as Example 1 and a suitable composition according to the present invention is as Examples 2 to 4, as follows:

| **FUNCTION** | **INGREDIENT** | **EX. 1 (%)** | **EX. 2 (%)** | **EX. 3 (%)** | **EX. 4 (%)** |
|---|---|---|---|---|---|
| active compound | disodium EDTA | 0.914 | 1.094 | 2.3 | 1.3 |
| | triethanolamine | | | | |
| pigment | titanium dioxide | 9.95 | 9.95 | 10.31 | 13,75 |
| | iron oxides | | | | |
| | iron oxides | | | | |
| | iron oxides | | | | |
| polymer | aluminum starch octenyl succinate | 6.2 | 5.4 | 4.18 | 7.0 |
| | xanthan gum | | | | |
| | acrylates/C₁₀₋₃₀ alkyl acrylate crosspolymer | | | | |
| | styrene/acrylates copolymer | | | | |
| UV filter | butyl methoxydibenzoylmethane | 30 | 30 | 20.5 | 30.3 |
| | ethylhexyl salicylate | | | | |
| | titanium dioxide | | | | |
| | octocrylene | | | | |
| | homosalate | | | | |
| | drometrizole trisiloxane | | | | |
| surfactant | sodium methyl stearoyl taurate | - | 0.5 | 5 | 2.5 |
| | potassium cetyl phosphate | 2 | 2 | 1 | 1.5 |
| | inulin lauryl carbamate | 1.3 | 1.3 | 3.5 | 6.5 |
| other | phenoxyethanol | 7.78 | 7.78 | 6.5 | 8.0 |
| | dimethicone | | | | |
| | caprylyl methicone | | | | |
| | C₁₂₋₁₅ alkyl benzoate | | | | |
| | caprylyl glycol | | | | |
| | silica | | | | |
| | tocopherol | 0,1 | 0.1 | 0.5 | 0.1 |
| water | water | Q.S. | Q.S. | Q.S. | Q.S. |

### Example 5

In order to demonstrate the superior stability of the composition according to the present invention, a test was performed with the composition of Example 2 (invention) and a composition according to Example 1 (prior art), wherein the compositions were analyzed after 30 and 60 days at specific temperatures (20ºC, 37ºC and 45ºC).

The results of the composition of the present invention show a perfect stability of the ingredients, with no phase separation at 20ºC (FIG. 1 (a)), at 37ºC (FIG. 1 (b)) and at 45ºC (FIG. 1 (c)) after 30 days. No phase separation was also observed after 60 days at 20ºC (FIG. 1 (d)), at 37ºC (FIG. 1 (e)) and at 45ºC (FIG. 1 (f)), and after a sun test (FIG. 1 (g)).

It can also be observed that the results for the composition according to the Example 1 (prior art) show that the composition is unstable after 30 days at 20ºC (FIG. 2 (a)), at 37ºC (FIG. 2 (b)) and at 45ºC (FIG. 2 (c)), and after 60 days at 20ºC (FIG. 2 (d)), at 37ºC (FIG. 2 (e)) and at 45ºC (FIG. 2 (f)).

### Example 6

In order to demonstrate the superior and robust compatibility of the ingredients of the composition according to Example 2 (invention), in view of Example 1 (prior art), a compatibility test was performed during 2 months. The compositions were evaluated at 45ºC.

The objective of the compatibility test is to analyze the stability of the composition inside the final package of the product (that is, on the package in which the product will be bottled for sale to the consumers; how the product will actually be on the market), considering how the composition will be at the top (sale position) and at the bottom (reverse position) of the package.

Based on the comparison of such compositions, it was possible to observe a superior compatibility of the ingredients and robustness of the composition of the present invention (comprising the surfactant system) both at the top of the package (sale position) and at the bottom of the package (reverse position), as showed by FIG. 3, in view of the composition of the state of the art (without the surfactant system), in both positions, as showed by FIG. 4.

### Example 7

A test was performed in order to evaluate the cutaneous safety of the composition of Example 2.

The product was used during 28 days by 50 adult subjects (between 18 and 66 years old), male and female, phototype III and IV, white and black, under dermatological control.

All subjects were considered as sensitive face skin.

After 28 days of product use, subjects were requested to return to the research institute, where the dermatologist performed a final cutaneous examination.

As a conclusion, the composition according to Example 2 is cutaneous safe.

### Example 8

A non-limiting example regarding the preparation of the composition of Examples 2 to 4, could be as follows:
Step (A): the oil phase comprising the oil raw materials is mixed, and heated up to 75ºC;
Step (B): the aqueous phase comprising the aqueous raw materials is mixed until complete homogenization, and heated up to 75ºC;
Step (C): the aqueous phase of step (B) is added to the oil phase of step (A), after which the emulsifier is subsequently added, followed by mixing the mixture;
Step (D): the polymers are added to the mixture obtained in step (C), subsequently followed by a neutralization;
Step (E): the fillers are gradually added to the mixture obtained in step (D) and mixed until homogeneity, at a temperature of below 30ºC.

## Claims

1. A sunscreen composition comprising:
(a) a combination of at least one anionic surfactant and one non-ionic co-surfactant selected from sodium N-stearoyl-N-methyl taurate and inulin lauryl carbamate respectively;
(b) UV filters; and
(c) pigments,
wherein the amount of the combination of at least one anionic surfactant and one non-ionic co-surfactant is ranging from about 0.5% to 10% by weight, including all ranges and sub-ranges there between, relative to the total weight of the composition.

2. Sunscreen composition, according to the preceding claim, wherein the amount of the combination of at least one anionic surfactant and one non-ionic co-surfactant is ranging from about 1.5% to about 8% by weight, preferably from about 3% to about 6% by weight, including all ranges and sub-ranges there between, relative to the total weight of the composition.

3. Sunscreen composition, according to any one of the preceding claims, wherein the combination of at least one anionic surfactant and one non-ionic co-surfactant employs a surfactant in an amount ranging from about 0.01% to about 7% by weight, preferably from about 0.3% to about 5% by weight, and more preferably from about 0.5% to about 3% by weight, including all ranges and sub-ranges there between, relative to the total weight of the composition.

4. Sunscreen composition, according to any one of the preceding claims, wherein the combination of at least one anionic surfactant and one non-ionic co-surfactant employs a co-surfactant in an amount ranging from about 0.1% to about 7% by weight, preferably from about 0.1% to about 6% by weight, and more preferably from about 0.2% to about 3% by weight, including all ranges and sub-ranges therebetween, relative to the total weight of the composition.

5. Sunscreen composition, according to any one of the preceding claims, wherein the UV filters are selected from oil-soluble organic ingredient, water-soluble organic sunscreen ingredient and silica-coated titanium dioxide sunscreen ingredient.

6. Sunscreen composition, according to any one of the preceding claims, wherein the UV filters in the sunscreen composition of the invention ranges from about 3% to about 50% by weight, preferably from about 5% to about 25% by weight, and more preferably from about 7% to about 18% by weight, based on the total weight of the composition.

7. Sunscreen composition, according to any one of the preceding claims, wherein the pigments are selected from the group of metal oxide pigments, such as titanium dioxide, zinc oxide, titanium oxide, iron oxide, zirconium oxide and cerium oxide, or mixtures thereof.

8. Sunscreen composition, according to any one of the preceding claims, wherein the amount of the pigments in the sunscreen composition of the invention ranges from about 0.01% to about 15% by weight, preferably from about 0.1% to about 5% by weight, and more preferably from about 0.5% to about 4% by weight, based on the total weight of the composition.

9. Sunscreen composition, according to any one of the preceding claims, wherein the pH of the sunscreen composition is within the range of about 5.5 to about 7.0, preferably within the range of about 6.0 to about 6.5.

10. Sunscreen composition, according to any one of the preceding claims, wherein it further comprises cosmetically acceptable ingredients selected from additional sunscreens, perfume/fragrance, preserving agents, solvents, actives, surfactants, fatty compounds, vitamins, fillers, silicones, polymers, pigments and mixtures thereof.

11. Sunscreen composition, according to any one of the preceding claims, wherein it presents a Sun Protection Factor 50, preferably a Sun Protection Factor 60, more preferably a Sun Protection Factor 70, and better a Sun Protection Factor 80.

12. Sunscreen composition, according to any one of the preceding claims, wherein it is in the form of an O/W emulsion.

13. Use of the sunscreen composition, as defined in any one of claims 1 to 12, wherein it is for the manufacture of a product to be used as sunscreen daily product.

14. Use, according to claim 13, wherein it is for the manufacture of a product which presents a high level of UV-protection in order to protect the skin from the damages of the sun, easy application, good spreadability, less white film, less shine, which does not melt on high temperature in the face, has an imperceptible touch and combines high protection with smoothness to the skin and is stable over the time.

15. Use, according to claim 13 or 14, wherein the composition is in the form of an O/W emulsion.

## Patentansprüche

1. Sonnenschutzzusammensetzung, die Folgendes umfasst:
(a) eine Kombination aus mindestens einem anionischen Tensid und einem nichtionischen Co-Tensid, ausgewählt aus Natrium-N-stearoyl-N-methyltaurat bzw. Inulinlaurylcarbamat;
(b) UV-Filter; und
(c) Pigmente,
wobei die Menge der Kombination aus mindestens einem anionischenTensid und einem nichtionischen Co-Tensid im Bereich von 0,5 Gew.-% bis 10 Gew.-% liegt, einschließlich aller Bereiche und Unterbereiche dazwischen, bezogen auf das Gesamtgewicht der Zusammensetzung.

2. Sonnenschutzzusammensetzung nach dem vorhergehenden Anspruch, wobei die Menge der Kombination aus mindestens einem anionischen Tensid und einem nichtionischen Co-Tensid im Bereich von etwa 1,5 Gew.-% bis etwa 8 Gew.-%, vorzugsweise von etwa 3 Gew.-% bis etwa 6 Gew.-% liegt, einschließlich aller Bereiche und Unterbereiche dazwischen, bezogen auf das Gesamtgewicht der Zusammensetzung.

3. Sonnenschutzzusammensetzung nach einem der vorhergehenden Ansprüche, wobei die Kombination aus mindestens einem anionischen Tensid und einem nichtionischen Co-Tensid ein Tensid in einer Menge im Bereich von etwa 0,01 Gew.-% bis etwa 7 Gew.-%, vorzugsweise von etwa 0,3 Gew.-% bis etwa 5 Gew.-% und mehr bevorzugt von etwa 0,5 Gew.-% bis etwa 3 Gew.-% verwendet, einschließlich aller Bereiche und Unterbereiche dazwischen, bezogen auf das Gesamtgewicht der Zusammensetzung.

4. Sonnenschutzzusammensetzung nach einem der vorhergehenden Ansprüche, wobei die Kombination aus mindestens einem anionischen Tensid und einem nichtionischen Co-Tensid ein Co-Tensid in einer Menge im Bereich von etwa 0,1 Gew.-% bis etwa 7 Gew.-%, vorzugsweise von etwa 0,1 Gew.-% bis etwa 6 Gew.-% und mehr bevorzugt von etwa 0,2 Gew.-% bis etwa 3 Gew.-% verwendet, einschließlichlich aller Bereiche und Unterbereiche dazwischen, bezogen auf das Gesamtgewicht der Zusammensetzung.

5. Sonnenschutzzusammensetzung nach einem der vorhergehenden Ansprüche, wobei die UV-Filter ausgewählt sind aus einem öllöslichen organischen Bestandteil, einem wasserlöslichen organischen Sonnenschutzbestandteil und einem Sonnenschutzbestandteil aus silikabeschichtetem Titandioxid.

6. Sonnenschutzzusammensetzung nach einem der vorhergehenden Ansprüche, wobei die UV-Filter in der erfindungsgemäßen Sonnenschutzzusammensetzung in einer Menge im Bereich von etwa 3 Gew.-% bis etwa 50 Gew.-%, vorzugsweise von etwa 5 Gew.-% bis etwa 25 Gew.-% und mehr bevorzugt von etwa 7 Gew.-% bis etwa 18 Gew.-% vorliegen, bezogen auf das Gesamtgewicht der Zusammensetzung.

7. Sonnenschutzzusammensetzung nach einem der vorhergehenden Ansprüche, wobei die Pigmente aus der Gruppe der Metalloxidpigmente ausgewählt sind, wie zum Beispiel Titandioxid, Zinkoxid, Titanoxid, Eisenoxid, Zirconoxid und Ceroxid, oder Mischungen davon.

8. Sonnenschutzzusammensetzung nach einem der vorhergehenden Ansprüche, wobei die Menge der Pigmente in der erfindungsgemäßen Sonnenschutzzusammensetzung im Bereich von etwa 0,01 Gew.-% bis etwa 15 Gew.-%, vorzugsweise von etwa 0,1 Gew.-% bis etwa 5 Gew.-% und mehr bevorzugt von etwa 0,5 Gew.-% bis etwa 4 Gew.-% liegt, bezogen auf das Gesamtgewicht der Zusammensetzung.

9. Sonnenschutzzusammensetzung nach einem der vorhergehenden Ansprüche, wobei der pH-Wert der Sonnenschutzzusammensetzung im Bereich von etwa 5,5 bis etwa 7,0 liegt, vorzugsweise im Bereich von etwa 6,0 bis etwa 6,5.

10. Sonnenschutzzusammensetzung nach einem der vorhergehenden Ansprüche, wobei sie ferner kosmetisch annehmbare Bestandteile umfasst, die ausgewählt sind aus zusätzlichen Sonnenschutzmitteln, Parfum/Duftstoff, Konservierungsmitteln, Lösungsmitteln, Wirkstoffen, Tensiden, Fettverbindungen, Vitaminen, Füllstoffen, Siliconen, Polymeren, Pigmenten und Mischungen davon.

11. Sonnenschutzzusammensetzung nach einem der vorhergehenden Ansprüche, wobei sie einen Sonnenschutzfaktor 50, vorzugsweise einen Sonnenschutzfaktor 60, mehr bevorzugt einen Sonnenschutzfaktor 70 und besser noch einen Sonnenschutzfaktor 80 bietet.

12. Sonnenschutzzusammensetzung nach einem der vorhergehenden Ansprüche, wobei sie in Form einer Ö/W-Emulsion vorliegt.

13. Verwendung der Sonnenschutzzusammensetzung nach einem der Ansprüche 1 bis 12, wobei sie zur Herstellung eines Produkts dient, das als tägliches Sonnenschutzprodukt zu verwenden ist.

14. Verwendung nach Anspruch 13, wobei sie zur Herstellung eines Produkts dient, das ein hohes Maß an UV-Schutz bietet, um die Haut vor Schäden durch die Sonne zu schützen, leicht aufzutragen ist, gut zu verteilen ist, weniger weißen Film hinterlässt, weniger glänzt, bei hoher Temperatur im Gesicht nicht schmilzt, bei Berührung nicht wahrnehmbar ist und hohen Schutz mit einer glatten Haut kombiniert und über die Zeit stabil ist.

15. Verwendung nach Anspruch 13 oder 14, wobei die Zusammensetzung in Form einer Ö/W-Emulsion vorliegt.

## Revendications

1. Composition d'écran solaire comprenant :
(a) une combinaison d'au moins un tensioactif anionique et un co-tensioactif non ionique sélectionnés parmi le sodium N-stéaroyl-N-méthyl taurate et le carbamate d'inuline laurylique respectivement ;
(b) des filtres UV ; et
(c) des pigments,
dans laquelle la quantité de la combinaison d'au moins un tensioactif anionique et un co-tensioactif non ionique se situe dans la plage d'environ 0,5 % à 10 % en poids, comportant toutes les plages et sous-plages entre elles, par rapport au poids total de la composition.

2. Composition d'écran solaire, selon la revendication précédente, dans laquelle la quantité de la combinaison d'au moins un tensioactif anionique et un co-tensioactif non ionique se situe dans la plage d'environ 1,5 % à environ 8 % en poids, de préférence d'environ 3 % à environ 6 % en poids, comportant toutes les plages et sous-plages entre elles, par rapport au poids total de la composition.

3. Composition d'écran solaire, selon l'une quelconque des revendications précédentes, dans laquelle la combinaison d'au moins un tensioactif anionique et un co-tensioactif non ionique utilise un tensioactif dans une quantité qui se situe dans la plage d'environ 0,01 % à environ 7 % en poids, de préférence d'environ 0,3 % à environ 5 % en poids, et de préférence encore d'environ 0,5 % à environ 3 % en poids, comportant toutes les plages et sous-plages entre elles, par rapport au poids total de la composition.

4. Composition d'écran solaire, selon l'une quelconque des revendications précédentes, dans laquelle la combinaison d'au moins un tensioactif anionique et un co-tensioactif non ionique utilise un co-tensioactif dans une quantité qui se situe dans une plage d'environ 0,1 % à environ 7 % en poids, de préférence d'environ 0,1 % à environ 6 % en poids, et de préférence encore d'environ 0,2 % à environ 3 % en poids, comportant toutes les plages et sous-plages entre elles, par rapport au poids total de la composition.

5. Composition d'écran solaire, selon l'une quelconque des revendications précédentes, dans laquelle les filtres UV sont sélectionnés à partir d'un ingrédient organique soluble dans l'huile, d'un ingrédient d'écran solaire organique soluble dans l'eau, et d'un ingrédient d'écran solaire de dioxyde de titane revêtu de silice.

6. Composition d'écran solaire, selon l'une quelconque des revendications précédentes, dans laquelle les filtres UV dans la composition d'écran solaire de l'invention se situent dans la plage d'environ 3 % à environ 50 % en poids, de préférence d'environ 5 % à environ 25 % en poids, et de préférence encore d'environ 7 % à environ 18 % en poids, sur la base du poids total de la composition.

7. Composition d'écran solaire, selon l'une quelconque des revendications précédentes, dans laquelle les pigments sont sélectionnés à partir du groupe de pigments d'oxyde de métal, tels que du dioxyde de titane, de l'oxyde de zinc, de l'oxyde de titane, de l'oxyde de fer, de l'oxyde de zirconium et de l'oxyde de cérium, ou des mélanges de ceux-ci.

8. Composition d'écran solaire, selon l'une quelconque des revendications précédentes, dans laquelle la quantité des pigments dans la composition d'écran solaire de l'invention se situe dans la plage d'environ 0,01 % à environ 15 % en poids, de préférence d'environ 0,1 % à environ 5 % en poids, et de préférence encore d'environ 0,5 % à environ 4 % en poids, sur la base du poids total de la composition.

9. Composition d'écran solaire, selon l'une quelconque des revendications précédentes, dans laquelle le pH de la composition d'écran solaire se situe dans la plage d'environ 5,5 à environ 7,0, de préférence dans la plage d'environ 6,0 à environ 6,5.

10. Composition d'écran solaire, selon l'une quelconque des revendications précédentes, dans laquelle elle comprend en outre des ingrédients acceptables sur le plan cosmétique sélectionnés à partir d'écrans solaires supplémentaires, de parfums/fragrances, de conservateurs, de solvants, d'actifs, de tensioactifs, de composés gras, de vitamines, d'agents de remplissage, de silicones, de polymères, de pigments et de mélanges de ceux-ci.

11. Composition d'écran solaire, selon l'une quelconque des revendications précédentes, dans laquelle elle présente un indice de protection solaire 50, de préférence un indice de protection solaire 60, de préférence encore un indice de protection solaire 70, et mieux un indice de protection solaire 80.

12. Composition d'écran solaire, selon l'une quelconque des revendications précédentes, dans laquelle elle est sous la forme d'une émulsion huile-dans-eau.

13. Utilisation de la composition d'écran solaire, telle que définie dans l'une quelconque des revendications 1 à 12, dans laquelle elle est destinée à la fabrication d'un produit devant être utilisé comme un produit de protection solaire quotidien.

14. Utilisation, selon la revendication 13, dans laquelle elle est destinée à la fabrication d'un produit qui présente un haut niveau de protection UV afin de protéger la peau des dommages du soleil, une application facile, une aptitude à l'étalement satisfaisante, moins de film blanc, moins de brillance, qui ne coule pas lors d'une température élevée sur le visage, possède un toucher imperceptible et associe une protection élevée à une sensation de douceur sur la peau et est stable dans le temps.

15. Utilisation, selon la revendication 13 ou 14, dans laquelle la composition est sous la forme d'une émulsion huile-dans-eau.
